(19) Europäisches Patentamt
European Patent Office
Office européen des brevets

(11) Veröffentlichungsnummer: **0 399 278 A2**

# (12) EUROPÄISCHE PATENTANMELDUNG

(21) Anmeldenummer: **90108575.3**

(22) Anmeldetag: **07.05.90**

(51) Int. Cl.5: **C07D 209/08, C07D 215/06, C08K 5/3417, C08K 5/3437**

(30) Priorität: **20.05.89 DE 3916494**

(43) Veröffentlichungstag der Anmeldung: **28.11.90 Patentblatt 90/48**

(84) Benannte Vertragsstaaten: **CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Spang, Peter, Dr.**
**Zur Audell 43**
**D-6670 St. Ingbert(DE)**
Erfinder: **Neumann, Peter, Dr.**
**Poststrasse 28**
**D-6800 Mannheim 31(DE)**
Erfinder: **Trauth, Hubert**
**Milanstrasse 6**
**D-6724 Dudenhofen(DE)**

## (54) Heterocyclische Amidinderivate.

(57) Heterocyclische Amidinderivate der allgemeinen Formel I

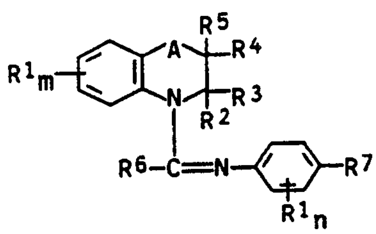

I

in der die Variablen

$R^1$ gleich oder verschieden sind und für Wasserstoff, $C_1$-$C_4$-Alkylreste, $C_1$-$C_4$-Alkoxyreste, Hydroxylgruppen oder Chloratome stehen,

$R^2$ bis $R^5$ Wasserstoff oder $C_1$-$C_4$-Alkylgruppen bedeuten, wobei $R^3$ und $R^4$ auch zu einem fünf- oder sechsgliedrigen Ring verbunden sein können,

$R^6$ für Wasserstoff oder eine $C_1$-$C_8$-Alkylgruppe steht,

$R^7$ eine Cyanogruppe oder einen der Reste $-COOR^8$, $-COR^8$, $-CONHR^8$ oder $-CONR^8{}_2$ bezeichnet, wobei

$R^8$ eine gradkettige oder verzweigte $C_1$-$C_{12}$-Alkylgruppe, die durch Sauerstoffatome unterbrochen sein kann, eine Cyclopentyl- oder Cyclohexylgruppe oder eine Phenylalkylgruppe mit 1 bis 3 C-Atomen in Alkylteil bedeutet,

A eine chemische Bindung oder eine Methylengruppe, die noch ein oder zwei $C_1$-$C_4$-Alkylreste tragen kann, bedeutet und

m und n jeweils für 1 oder 2 stehen.

Die heterocyclischen Amidinderivate I dienen als Lichtschutzmittel für organische Materialien.

EP 0 399 278 A2

## Heterocyclische Amidinderivate

Die vorliegende Erfindung betrifft neue heterocyclische Amidinderivate der allgemeinen Formel I

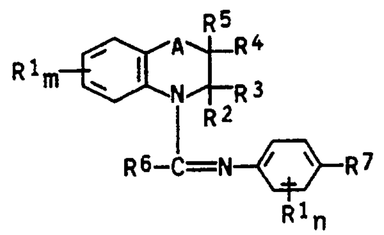

I

in der die Variablen

$R^1$ gleich oder verschieden sind und für Wasserstoff, $C_1$-$C_4$-Alkylreste, $C_1$-$C_4$-Alkoxyreste, Hydroxylgruppen oder Chloratome stehen,

$R^2$ bis $R^5$ Wasserstoff oder $C_1$-$C_4$-Alkylgruppen bedeuten, wobei $R^3$ und $R^4$ auch zu einem fünf- oder sechsgliedrigen Ring verbunden sein können,

$R^6$ für Wasserstoff oder eine $C_1$-$C_8$-Alkylgruppe steht,

$R^7$ eine Cyanogruppe oder einen der Reste $-COOR^8$, $-COR^8$, $-CONHR^8$ oder $-CONR^8_2$ bezeichnet, wobei

$R^8$ eine gradkettige oder verzweigte $C_1$-$C_{12}$-Alkylgruppe, die durch Sauerstoffatome unterbrochen sein kann, eine Cyclopentyl- oder Cyclohexylgruppe oder eine Phenylalkylgruppe mit 1 bis 3 C-Atomen in Alkylteil bedeutet,

A eine chemische Bindung oder eine Methylengruppe, die noch ein oder zwei $C_1$-$C_4$-Alkylreste tragen kann, bedeutet und

m und n jeweils für 1 oder 2 stehen.

Außerdem betrifft die Erfindung ein Verfahren zur Herstellung dieser Verbindungen, ihre Verwendung als Lichtschutzmittel für organische Materialien sowie gegen die Einwirkung von Licht stabilisierte organische Materialien, die diese Verbindungen enthalten.

Aus der US-A 4 021 471 sind offenkettige Formamidinderivate mit der Grundstruktur

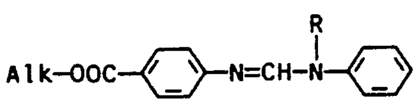

Alk = Alkyl
R = Alkyl, Phenyl

als UV-Stabilisatoren für organische Materialien bekannt.

Die Absorptionsmaxima der meisten dieser Verbindungen liegen jedoch im Bereich von 310 bis 320 nm (UV-B-Bereich). UV-Licht mit einer größeren Wellenlänge als 360 nm wird durch diese Verbindungen nicht mehr ausreichend absorbiert, so daß organische Materialien, beispielsweise Kunststoffe, die diese Mittel enthalten, in diesem Wellenlängenbereich (UV-A) praktisch nicht mehr gegen Schädigungen durch Lichteinwirkung geschützt werden. Weiterhin läßt die Photostabilität dieser Verbindungen bei Lichteinwirkung über einen längeren Zeitraum zu wünschen übrig.

Aufgabe der vorliegenden Erfindung war es daher, Lichtschutzmittel für organische Materialien bereitzustellen, die bei guter Verträglichkeit mit den zu schützenden Materialien und hoher Langzeit-Photostabilität bei größeren Wellenlängen größer als 360 nm auch noch eine hohe Wirksamkeit aufweisen.

Demgemäß wurden die eingangs definierten heterocyclischen Amidinderivate I gefunden.

Weiterhin stehen die Reste $R^1$ bevorzugt für Wasserstoff, $C_1$-$C_4$-Alkyl- oder $C_1$-$C_4$-Alkoxygruppen, insbesondere für Wasserstoff, Methyl- und Methoxygruppen.

Die Reste $R^2$ bis $R^5$ bezeichnen Wasserstoff oder $C_1$-$C_4$-Alkylgruppen, insbesondere Methylgruppen. Weiterhin können $R^3$ und $R^4$ vorteilhafterweise zu einem fünf- oder sechsgliedrigen Ring verbunden sein.

Die Reste $R^1$ bis $R^5$ entsprechen den als Ausgangsverbindungen dienenden heterocyclischen Aminen III

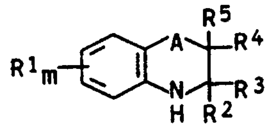

III

unter denen die folgenden Verbindungen im Hinblick auf die Eigenschaften von I besonders bevorzugt sind:
Indolin
2-Methylindolin
3-Methylindolin
4-Methylindolin
7-Methylindolin
2,3-Dimethylindolin
3,3-Dimethylindolin
2,5-Dimethylindolin
2,7-Dimethylindolin
2,3,3-Trimethylindolin
2,3,7-Trimethylindolin
2,2,3,3-Tetramethylindolin
2,3,3-Trimethyl-5-methoxyindolin
1,2,3,4-Tetrahydrochinolin
1,2,3,4-Tetrahydrochinaldin
3-Methyl-1,2,3,4-tetrahydrochinolin
4-Methyl-1,2,3,4-tetrahydrochinolin
5-Methyl-1,2,3,4-tetrahydrochinolin
6-Methyl-1,2,3,4-tetrahydrochinolin
7-Methyl-1,2,3,4-tetrahydrochinolin
1,2,3,4,10,11-Hexahydrocarbazol

Der Rest $R^6$ bedeutet vorzugsweise eine Methyl-, Ethyl-, n-Propyl- oder iso-Propylgruppe oder insbesondere Wasserstoff.

Der Rest $R^7$ steht bevorzugt für eine Cyanogruppe oder den Rest -$COOR^8$.

Der Rest $R^8$ steht vorzugsweise für eine gradkettige oder verzweigte, gegebenenfalls durch Sauerstoffatome unterbrochene $C_1$-$C_{12}$-Alkylgruppe. Bevorzugte Reste $R^8$ sind die Methyl-, Ethyl-, n-Propyl-, iso-Propyl-, n-Butyl-, iso-Butyl-, sek.-Butyl-, tert.-Butyl-, n-Pentyl-, n-Hexyl-, n-Heptyl-, 2-Ethylhexyl-, n-Octyl, n-Nonyl-, iso-Nonyl-, n-Decyl-, n-Dodecyl-, 2-Methoxyethyl-, 2-Ethoxyethyl- und 2-Butoxyethylgruppe sowie die Gruppen
$-CH_2CH_2OCH_2CH_2OC_2H_5$,
$-CH_2CH_2OCH_2CH_2OC_4H_9$,
$-CH_2CH_2OCH_2CH_2OCH_2CH_2OC_2H_5$,
$-CH_2CH_2OCH_2CH_2OCH_2CH_2OC_4H_9$
$-CH_2CH_2OCH_2CH_2OCH_2CH_2OCH_2CH_2OC_2H_5$ und
$-CH_2CH_2OCH_2CH_2OCH_2CH_2OCH_2CH_2OC_4H_9$.

Ganz besonders bevorzugt sind von den Verbindungen I solche der allgemeinen Formel Ia, wobei A insbesondere eine chemische Bindung oder die Gruppierung -$CH_2$- oder -$CH(CH_3)$- bezeichnet

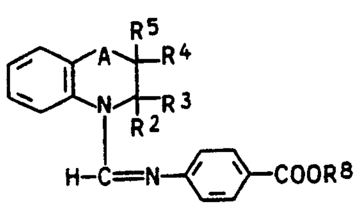

Ia

Die erfindungsgemäßen heterocyclischen Amidinderivate I stellt man zweckmäßigerweise durch Umsetzung von N-Arylimidaten II

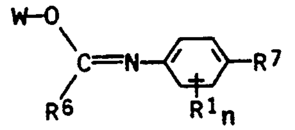

II

bei denen W für einen $C_1$-$C_4$-Alkylrest wie Methyl, Ethyl oder Propyl steht, mit heterocyclischen Aminen III in an sich bekannter Weise her.

Die N-Arylimidate II lassen sich nach bekannten Methoden, beispielsweise aus den entsprechenden Anilinderivaten V und Orthoalkansäuretrialkylestern, herstellen.

Für den Fall, daß $R^6$ Wasserstoff bedeutet, ist es auch möglich, die erfindungsgemäßen heterocyclischen Amidinderivate I vorteilhaft durch Umsetzung von heterocyclischen N-Formylaminen IV

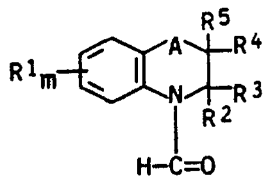

IV

mit Anilinderivaten V

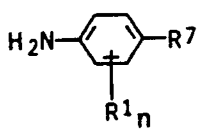

V

in an sich bekannter Weise herzustellen.

Die N-Formylamine IV sind leicht nach bekannten Methoden, beispielsweise durch Formylierung der heterocyclischen Amine III, zugänglich.

Die Derivate der allgemeinen Formeln I und Ia, in denen $R^8$ für einen höheren, gegebenenfalls durch Sauerstoffatome unterbrochenen Alkylrest oder einen Phenylalkylrest steht, lassen sich auch gut durch Umesterung der niederen Ester, bei denen $R^8$ zum Beispiel eine Methyl- oder Ethylgruppe bezeichnet, in an sich bekannter Weise herstellen.

Die erfindungsgemäßen heterocyclischen Amidinderivate I dienen als Lichtschutzmittel für organische Materialien, beispielsweise für kosmetische Präparate, für Arzneimittelformulierungen oder für Vorprodukte für Kunststoffe, insbesondere jedoch für Kunststoffe selbst.

Als Kunststoffe kommen insbesondere Polyurethane, Polyester, Polyamide, Polystyrol, Acrylsäure-Butadien-Styrol-Copolymere, heißvernetzbare Acrylharze und thermoplastische Acrylharze in Betracht, daneben aber auch Polyolefine, halogenhaltige Vinylpolymere, Polyacrylate, Polymethacrylate, Polyacrylamide, Polyacrylnitril, Polyvinylalkohol und dessen Acylderivate, Polyacetate, Polyalkylenoxide, Polyphenylenoxide, Polyharnstoffe, Polysulfone, Polycarbonate, vernetzte Polymere aus Aldehyden und Phenolen, Harnstoff oder Melamin, ungesättigte Polyesterharze und Alkydharze.

Die erfindungsgemäßen heterocyclischen Amidinderivate I eignen sich besonders gut zum Schutz von Polyurethanen, wobei es sich empfiehlt, diese Mittel bereits den Polyurethan-Komponenten, also der Polyol-Komponente oder der Isocyanat-Komponente, zuzusetzen. Im Falle der so stabilisierten Polyurethane ist nicht nur eine gute Lichtschutzwirkung, sondern auch eine bessere Wärmestabilität zu beobachten.

Gegenstand dieser Erfindung sind außerdem gegen die Einwirkung von Licht stabilisierte organische Materialien, instiesondere Kunststoffe, welche, bezogen auf die Menge des organischen Materials, 0,01 bis 10 Gew.%, vorzugsweise 0,1 bis 5 Gew.% eines oder mehrerer heterocyclischer Amidinderivate I enthalten.

Außer den Verbindungen I können die organischen Materialien noch weitere Hilfs- und Füllstoffe in den hierfür üblichen Mengen enthalten, beispielsweise:

- weitere Lichtschutzmittel wie 2-(2′-Hydroxyphenyl)benztriazole, 2,4-Bis(2′-hydroxyphenyl)-6-alkyl-s-triazine, 2-Hydroxybenzophenone oder 1,3-Bis(2′-hydroxybenzoyl)benzole
- Antioxidantien auf der Basis von Phosphor- und Schwefelverbindungen und von sterisch gehinderten Phenolen und Aminen
- Füllstoffe wie Ruß, Kaolin, Talk oder Glasfasern
- Pigmente wie Titandioxid

- Sonstige Zusatzstoffe wie Weichmacher, Gleitmittel, Emulgatoren, optische Aufheller, Flammschutzmittel oder Antistatica.

Bei Verwendung der genannten Antioxidantien kann die stabilisierende Wirkung gegen die Einwirkung von Licht und auch gegen thermische Belastung noch deutlich gesteigert werden. Diese Mittel oder Gemische davon werden zweckmäßigerweise in einer Menge von 0,1 bis 5 Gew.%, vorzugsweise 0,5 bis 3 Gew.%, bezogen auf die Menge des organischen Materials, zusammen mit den erfindungsgemäßen Verbindungen I eingesetzt.

Als phosphorhaltige Antioxidantien sind z.B.
Tris(nonylphenyl)phosphit,
Distearylpentaerythritdiphosphit,
Tris(2,4-di-tert.-butylphenyl)phosphit,
Tris(2-tert.-butyl-4-methylphenyl)phosphit,
Bis(2,4-di-tert.-butylphenyl)pentaerythritdiphosphit und
Tetrakis(2,4-di-tert.-butylphenyl)-4,4′-biphenylendiphosphit
zu nennen.

Als schwefelhaltige Antioxidationsmittel sind z.B.
Dilaurylthiodipropionat,
Dimyristylthiodipropionat,
Distearylthiodipropionat,
Pentaerythrittetrakis(β-laurylthiopropionat) und
Pentaerythrittetrakis (β-hexylthiopropionat)
zu nennen.

Als sterisch gehinderte phenolische Antioxidationsmittel kommen z.B. 2,6-Di-tert.-butyl-4-methylphenol,
n-Octadecyl-β-(3,5-di-tert.-butyl-4-hydroxyphenyl)propionat,
1,1,3-Tris(2-methyl-4-hydroxy-5-tert.-butylphenyl)butan,
1,3,5-Trimethyl-2,4,6-tris(3,5-di-tert.-butyl-4-hydroxybenzyl)benzol,
1,3,5-Tris(3,5-di-tert.-butyl-4-hydroxybenzyl)isocyanurat,
1,3,5-Tris[(3,5-di-tert.-butyl-4-hydroxyphenyl)propionyloxy-ethyl]isocyanurat,
1,3,5-Tris(2,6-di-methyl-3-hydroxy-4-tert.-butylbenzyl)isocyanurat,
Pentaerythrit-tetrakis[β-(3,5-di-tert.-butyl-4-hydroxyphenyl)propionat] sowie Tocopherol
in Betracht.

Als sterisch gehinderte Amine kommen z.B.
Bis(2,2,6,6-tetramethylpiperidyl)sebacat,
Bis(1,2,2,6,6-pentamethylpiperidyl)sebacat,
das Kondensationsprodukt von 1-Hydroxyethyl-2,2,6,6-tetramethyl-4-hydroxypiperidin und Bernsteinsäure,
das Kondensationsprodukt von N,N′-(2,2,6,6-Tetramethylpiperidyl)hexamethylendiamin und 4-tert.-Octylamino-2,6-dichlor-1,3,5-s-triazin,
Tris(2,2,6,6-tetramethylpiperidyl)nitrilotriacetat,
Tetrakis(2,2,6,6-tetramethyl-4-piperidyl)-1,2,3,4-butan-tetracarbonsäure,
1,1′-(1,2-Ethandiyl)-bis(3,3,5,5-tetramethylpiperzinon) und
die Kondensationsprodukte von 4-Amino-2,2,6,6,-tetramethylpiperidinen und
Tetramethylol-acetylendiharnstoffen
in Betracht.

Eine ganz besonders gute Stabilisierungswirkung erhält man im Falle von Polyurethanen, wenn das Polyurethan mit einem Gemisch aus einem erfindungsgemäßen heterocyclischen Amidinderivat I, einem sterisch gehinderten Phenol oder einer der genannten Schwefel- oder Phosphorverbindungen und einem sterisch gehinderten Amin stabilisiert wird.

Die erfindungsgemäßen heterocyclischen Amidinderivate I sind praktisch farblos und haben in der Regel Absorptionsmaxima im Bereich von 320 bis 350 nm und ausreichend hohe Extinktionskoeffizienten, so daß das Licht des UV-A-Bereichs, insbesondere mit einer größeren Wellenlänge als 360 nm, durch diese Verbindungen noch ausreichend absorbiert wird. Als weitere Vorteile sind die hohe Photostabilität der erfindungsgemäßen Verbindungen I bei Lichteinwirkung über einen längeren Zeitraum und die gute Verträglichkeit mit den zu stabilisierenden organischen Materialien, insbesondere mit Kunststoffen, anzuführen.

<u>Herstellungsbeispiele für heterocyclische Amidinderivate Ia</u>

Beispiele 1 bis 3 nach Methode (a)

0,25 mol eines N-Arylformimidates II

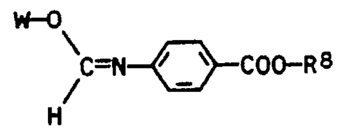

wurden bei T° C im Laufe von t Stunden mit 0,27 mol eines heterocyclischen Amins III

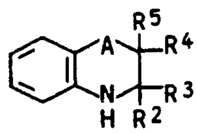

umgesetzt, wobei freiwerdender Alkohol abdestilliert wurde. Nach dem Abkühlen auf ca. 80° C wurde das Umsetzungsprodukt mit p ml Methanol zur Kristallisation gebracht und nochmals aus L umkristallisiert.

Beispiele 4 und 5 nach Methode (b)

0,25 mol eines in 250 ml Chloroform gelösten N-Formylamins IV

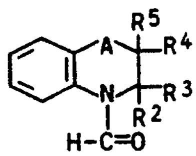

wurden zuerst mit 0,25 mol Phosphoroxitrichlorid bei T° C im Laufe von t Stunden und danach mit 0,25 mol eines Anilinderivates V

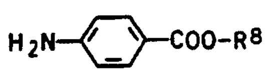

bei T′° C im Laufe von t′ Stunden umgesetzt. Nach dem Abkühlen wurde die Reaktionsmischung durch Eingießen in eine Natronlauge-Eis-Mischung, Phasentrennung, Abdestillieren des Chloroforms und Umkristallisieren des Rückstandes aus L aufgearbeitet.

Beispiele 6 bis 8 nach Methode (c)

0,20 mol des jeweiligen Ethylesters Ia aus Beispiel 3 bzw. 1 bzw. 5 wurden mit 3 g $Na_2CO_3$ und p ml 2-Ethylhexanol unter Durchleiten von Stickstoff bei T° C t Stunden lang gerührt. Nach dem Abkühlen und Filtrieren wurde überschüssiges 2-Ethylhexanol im Vakuum abdestilliert, der Rückstand in Essigsäureethylester aufgenommen und mit Aktivkohle und Bleicherde behandelt. Nach Filtrieren und Abdestillieren des Lösungsmittels im Vakuum erhielt man jeweils ein blaßgelbes Öl.

Einzelheiten zu diesen Beispielen sowie zu den erhaltenden Produkten sind Tabelle 1 zu entnehmen.

Tabelle 1

Herstellung von heterocyclischen Amidinderivaten Ia

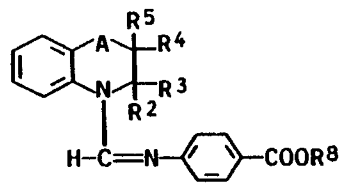

| Beispiel Nr. | A | $R^2$ | $R^3$ | $R^4$ | $R^5$ | $R^8$ | W | T [°C] | t [h] | T' [°C] | t' [h] | p (ml) | L | Ausbeute [g] | Schmelzpunkt [°C] | UV-Daten $\lambda_{max}$ [nm] | UV-Daten $\varepsilon(CH_2Cl_2)$ |
|---|---|---|---|---|---|---|---|---|---|---|---|---|---|---|---|---|---|
| Methode (a) | | | | | | | | | | | | | | | | | |
| 1 | - | H | H | H | H | $C_2H_5$ | $C_2H_5$ | 160 | 1,25 | | | 400 | Methanol | 45,9 | 101-102 | 338 | 31 000 |
| 2 | $CH_2$ | H | H | H | H | $C_2H_5$ | $C_2H_5$ | 190 | 2 | | | 125 | Methanol | 66,5 | 119-120 | 326 | 32 000 |
| 3 | - | $CH_3$ | H | H | H | $C_2H_5$ | $C_2H_5$ | 190 | 2 | | | 350 | Ethanol* | 58,8 | 103-105 | 338 | 31 600 |
| Methode (b) | | | | | | | | | | | | | | | | | |
| 4 | - | $CH_3$ | H | H | H | $C_2H_5$ | | 40 | 0,25 | 60 | 2 | | Ethanol* | 59,2 | wie Beispiel 3 | | |
| 5 | - | H | $-(CH_2)_4-$ | | H | $C_2H_5$ | | 40 | 2 | 60 | 2 | | Essigester* | 48,5 | 107-108 | 337 | 34 100 |

O.Z. 0050/40825

Tabelle 1 (Forts.)

| Beispiel Nr. | A | $R^2$ | $R^3$ | $R^4$ | $R^5$ | $R^8$ | W | T [°C] | t [h] | T' [°C] | t' [h] | p (ml) | L | Ausbeute [g] | Schmelzpunkt [°C] | UV-Daten $\lambda_{max}$ [nm] | UV-Daten $\varepsilon(CH_2Cl_2)$ |
|---|---|---|---|---|---|---|---|---|---|---|---|---|---|---|---|---|---|
| Methode (c) | | | | | | | | | | | | | | | | | |
| 6 | - | $CH_3$ | H | H | H | 2-Ethylhexyl | | 135 | 36 | | | 100 | | 74 | | 337 | 33 900 |
| 7 | - | H | H | H | H | 2-Ethylhexyl | | 135 | 23 | | | 235 | | 66,6 | | 338 | 31 200 |
| 8 | - | H | $-(CH_2)_4-$ | | H | 2-Ethylhexyl | | 130 | 15 | | | 255 | | 75,0 | | 338 | 32 200 |

*unter Aktivkohlezusatz

Anwendungsbeispiele

Beispiele 9 bis 12

Zur Herstellung von Belichtungsproben aus Polyurethan wurde eine Mischung aus
92,5 g einer Polyolkomponente der Zusammensetzung
41,9 g eines Polyetherols der OH-Zahl 29 mit ca. 84 % primären Hydroxylgruppen, erhalten durch Addition von Propylenoxid und Ethylenoxid an Polypropylenglykol,
42,5 g eines Polyetherols der OH-Zahl 27 mit ca. 88 % primären Hydroxylgruppen, erhalten durch Addition von Propylenoxid und Ethylenoxid an Trimethylolpropan, und
8,1 g 1,4-Butandiol,
1,7 g einer 25 gew.%igen Lösung von 1,4-Diazabicyclo[2.2.2]octan in 1,4-Butandiol,
0,02 g Dibutylzinndilaurat,
0,1 g eines handelsüblichen Siliconstabilisators,
5,5 g Fluortrichlormethan und
0,2 g Wasser
mit 0,5 g des jeweiligen erfindungsgemäßen heterocyclischen Amidinderivates aus den Beispielen 2, 5, 6 und 7 bzw. eines Amidines des Standes der Technik,
0,5 g Bis(1,2,2,6,6-pentamethylpiperidyl)sebacat sowie
0,25 g eines Antioxidans-Gemisches aus 9 Gew.% α-Tocopherol und 91 Gew.% Tris(nonylphenyl)phosphit versetzt
und mit 48,5 g eines 23 Gew.% Isocyanatgruppen enthaltenden Prepolymeren, hergestellt aus
87,2 Gew.% 4,4′-Diphenylmethandiisocyanat,
4,8 Gew.% eines Polyetherols der OH-Zahl 250, erhalten durch Addition von Propylenoxid an Propylenglykol, und
8,0 Gew.% Dipropylenglykol
bei 25 °C Komponenten- und Werkzeugtemperatur zu Prüfplatten verschäumt.

Die Prüfplatten wurden im Xenotest® 450 der Firma Hanau belichtet und an den Proben wurde der Yellowness Index (YI) gemäß Annual Book of ASTM Standards D 1925-70 (Reapproved 1977) als Maß für den Vergilbungsgrad bestimmt. Die Ergebnisse sind in der unten aufgeführten Tabelle 2 zusammengestellt.

Tabelle 2
YI-Werte von Polyurethan-Proben

| Beispiel Nr. | Stabilisator aus Beispiel | YI-Wert vor der Belichtung | Yi-Wert nach 96 h |
|---|---|---|---|
| erfindungsgemäß: | | | |
| 9 | 2 | 1,6 | 16,8 |
| 10 | 5 | 5,0 | 15,8 |
| 11 | 6 | 5,8 | 18,1 |
| 12 | 7 | 4,9 | 17,8 |
| zum Vergleich: | | | |
| ohne Stabilisator | | 5,0 | 39,9 |
| offenkettiges Amidin* | | 5,0 | 18,4 |

*Verbindung 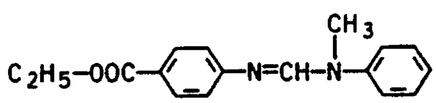 gemäß US-A 4 021 471

## Ansprüche

1. Heterocyclische Amidinderivate der allgemeinen Formel I

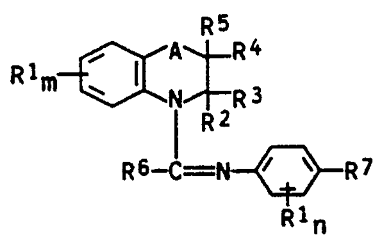

I

in der die Variablen
$R^1$ gleich oder verschieden sind und für Wasserstoff, $C_1$-$C_4$-Alkylreste, $C_1$-$C_4$-Alkoxyreste, Hydroxylgruppen oder Chloratome stehen,
$R^2$ bis $R^5$ Wasserstoff oder $C_1$-$C_4$-Alkylgruppen bedeuten, wobei $R^3$ und $R^4$ auch zu einem fünf- oder sechsgliedrigen Ring verbunden sein können,
$R^6$ für Wasserstoff oder eine $C_1$-$C_8$-Alkylgruppe steht,
$R^7$ eine Cyanogruppe oder einen der Reste $-COOR^8$, $-COR^8$, $-CONHR^8$ oder $-CONR^8{}_2$ bezeichnet, wobei
$R^8$ eine gradkettige oder verzweigte $C_1$-$C_{12}$-Alkylgruppe, die durch Sauerstoffatome unterbrochen sein kann, eine Cyclopentyl- oder Cyclohexylgruppe oder eine Phenylalkylgruppe mit 1 bis 3 C-Atomen in Alkylteil bedeutet,
A eine chemische Bindung oder eine Methylengruppe, die noch ein oder zwei $C_1$-$C_4$-Alkylreste tragen kann, bedeutet und
m und n jeweils für 1 oder 2 stehen.

2. Heterocyclische Amidinderivate I nach Anspruch 1, wobei die Variablen
$R^1$ gleich oder verschieden sind und für Wasserstoff, $C_1$-$C_4$-Alkylreste oder $C_1$-$C_4$-Alkoxyreste stehen,
$R^6$ Wasserstoff oder eine $C_1$-$C_3$-Alkylgruppe bedeutet und

$R^7$ eine Cyanogruppe oder den Rest -$COOR^8$ bezeichnet.

3. Verfahren zur Herstellung von heterocyclischen Amidinderivaten I gemäß Anspruch 1 oder 2, dadurch gekennzeichnet, daß man

(a) N-Arylimidate der allgemeinen Formel II

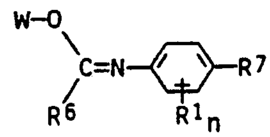

II

in der W für einen $C_1$-$C_4$-Alkylrest steht, mit heterocyclischen Aminen der allgemeinen Formel III

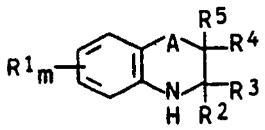

III

umsetzt oder

(b) für den Fall, daß $R^6$ Wasserstoff bedeutet, heterocyclische N-Formylamine der allgemeinen Formel IV

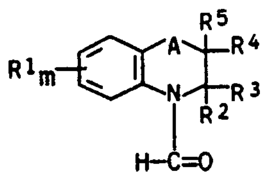

IV

mit Anilinderivaten der allgemeinen Formel V

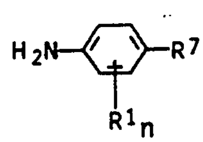

V

umsetzt.

4. Verwendung von heterocyclischen Amidinderivaten I gemäß Anspruch 1 oder 2 als Lichtschutzmittel für organische Materialien.

5. Gegen die Einwirkung von Licht stabilisierte organische Materialien, enthaltend 0,01 bis 10 Gew.%, bezogen auf die Menge des organischen Materials, eines heterocyclischen Amidinderivates I gemäß den Ansprüchen 1 oder 2.

6. Gegen die Einwirkung von Licht stabilisierte Kunststoffe, enthaltend 0,01 bis 10 Gew.%, bezogen auf die Menge des Kunststoffes, eines heterocyclischen Amidinderivates I gemäß den Ansprüchen 1 oder 2.